# EUROPEAN PATENT SPECIFICATION

(11) **EP 1 610 677 B1**
(45) Date of publication and mention of the grant of the patent: **28.11.2007**
(21) Application number: 04723996.7
(22) Date of filing: 29.03.2004
(51) Int. Cl.: A61B 5/0408, A61N 1/04

(54) **INTEGRATED STRUCTURE FOR DETECTING PHYSIOLOGICAL SIGNALS**
INTEGRIERTE STRUKTUR ZUM NACHWEIS VON PHYSIOLOGISCHEN SIGNALEN
STRUCTURE INTEGREE PERMETTANT DE DETECTER DES SIGNAUX PHYSIOLOGIQUES

(30) Priority: 01.04.2003 IT MI20030649
(43) Date of publication of application: 04.01.2006
(73) Proprietor: Fondazione Don Carlo Gnocchi - Onlus, 20121 Milano (IT)
(72) Inventor: DI RIENZO, Marco, I-20133 Milano (IT); PICCINI, Luca, I-20135 Milano (IT); CASTIGLIONI, Paolo, I-21100 Varese (IT); ANDREONI, Guiseppe, I-20053 Muggio' (IT); PEDOTTI, Antonio, I-20129 Milano (IT)
(74) Representative: Alagem Modiano, Lara S.
(86) International application number: PCT/EP2004/003319
(87) International publication number: WO 2004/086968

(56) References cited:
- WO-A-01/02052
- WO-A-02/40091
- US-A- 4 729 377
- US-A- 6 047 203

## Description

### Technical Field

The present invention relates generically to the acquisition of signals that are significant for monitoring physiological activities of a human being or of an animal in general.

### Background art

The acquisition and monitoring of physiological signals are useful and often necessary in many contexts, mostly in the sports or medical field. In the first case, typical example of monitoring of physiological signals linked to vital functions are electrocardiography, which allow to generate a trace of cardiac activity, and electromyography, which allows to monitor muscle functionality. The information derived from these acquisitions is essential in order to evaluate the health of the subject or patient.

As regards the sports field, it is often important to monitor the heartbeat rate of an athlete, both for safety reasons, for example in the case of prolonged workouts or stress tests, and in order to optimize the yield of physical activity.

One problem that often limits the use of instruments for monitoring physiological signals is the need to apply appropriate electrodes, with the corresponding connections to the signal collection device, directly on the body of the user, with obvious discomfort for the user. Moreover, in order to ensure adequate quality of the signal throughout the period of acquisition, conventional monitoring systems resort to gels or creams that increase the conductivity of the skin-electrode interface and to small suckers, clips, adhesive tape, elastic bandages and belts, in order to keep the electrodes in the correct position, causing further discomfort to the user.

Therefore, currently in cases in which there is the need to monitor the behavior of biological signals over an entire day, recording devices are used which store the data that arrive from electrodes that are appropriately positioned on the body of the human being, as occurs in the case of the so-called Holter test, but this forces the patient to prolonged and uncomfortable coexistence with sensors applied to his body, connecting wires and the data recording device.

The problems mentioned above may considerably limit the use of monitorings of biological signals, even in cases in which constant verification of the physical conditions of the subject would be useful. An attempt has been made therefore in the prior art to find more friendly methods for detecting physiological signals, so as to allow a more comfortable use of systems for monitoring vital functions.

In this context, PCT publication WO 01/02052 discloses a type of sensor, obtained by spinning conducting fibers, which is coupled to an item of clothing that can be worn by the user. This sensor, once placed in contact with the skin of the user, allows to transfer the acquired physiological signals to a data collection device by means of a number of connections provided within the item of clothing, thus eliminating the discomfort of direct positioning of a conventional electrode on the skin.

Likewise, PCT publication WO 02/40091 in the name of Georgia Tech discloses the use of sensors applied to an item of clothing, which are connected to a grid of conducting elements that carry the signal to a signal collection point within the item of clothing.

The solutions proposed in the prior art therefore allow the human being to wear an item of clothing provided with sensors that are sewn or applied to the fabric, thus partially reducing the discomfort that affects the user during the acquisition of physiological signals: however, even these solutions suffer important problems that considerably limit their potential use.

Specifically, these solutions are not capable of ensuring the acquisition of a physiological signal in a sufficiently strong and stable manner. During monitoring, particularly if performed for a prolonged time on users that can move freely, the electrodes may in fact not remain perfectly in contact with the skin. Moreover, due to the inevitable relative movements between the item of clothing and the body of the user, the electrodes, which are rigidly coupled to the item of clothing, may be displaced in positions that are not suitable for the acquisition of a given biological signal. In these cases there is a deterioration of quality or even a loss of the signal, obviously compromising the information content of the monitoring process.

### Disclosure of the Invention

The aim of the present invention is to provide a structure that allows even prolonged monitoring of physiological signals and overcomes the problems observed in the prior art.

Within this aim, an object of the present invention is to provide an integrated structure for acquiring physiological signals that allows to acquire physiological signals in a manner that is particularly comfortable for the user and at the same time is capable of acquiring the signals more reliably than the prior art while the user is performing his usual activities.

Another object of the present invention is to provide a system that is versatile and allows to acquire various kinds of physiological signal according to the requirements.

Another object of the present invention is to provide a system that allows real-time processing or post-processing of the data acquired during the signal monitoring period.

This aim and these and other objects that will become better apparent hereinafter are achieved by an integrated structure for acquiring physiological signals, comprising a fabric composed of conducting fibers and non-conducting fibers, a plurality of electrodes provided by means of conducting fibers, a plurality of conducting elements for connection between the electrodes and the inputs of a control device and the control device itself, which in turn comprises means for selective processing of electrical signals, particularly biological or physiological signals, that originate from the electrodes. The conductive elements are insulated one another and insulated by virtue of locally removable material so as to allow electrical coupling between two or more of said conducting elements and between the conducting elements and the skin of the user.

Conveniently, the integrated structure comprises means for selectively sending electrical signals to the electrodes.

The connecting elements are preferably provided by means of conducting fibers that are spun within the fabric or positioned on the fabric by affixing them, for example by gluing, embroidering or stitching.

Advantageously, the conducting fibers can be constituted by different conducting materials, for example different metals, so as to be able to utilize the physical properties of junctions between these materials for use as thermocouples, utilizing the principles and methods that are well-known in the art.

Moreover, each electrode can be adapted to detect and send a plurality of signals of different types, can be selected even dynamically within a wider group of available electrodes, and can be configured even dynamically so that it can acquire and send signals of a different type. Each electrode, moreover, can be arranged not only directly in contact with the skin of the user but also alternately in contact with the conducting surface of a conventional electrode, which in turn is arranged in contact with the skin of the user.

Conveniently, the control device comprises means for performing operations for configuring and selecting the electrodes both autonomously, according to preset programs, and under external control imparted by the user by acting directly on the control device or by means of a wired or wireless link, for example using radio frequencies or infrared rays, between the control device and external systems provided with independent intelligence.

Advantageously, the fabric of the integrated structure is provided in the form of an item of clothing or of any fabric that is placed in the environment and with which the subject can make contact during his activity. For example, it can be a sheet, a tablecloth, a sofa cover, a wrist support for a computer, and so forth.

Moreover, the integrated structure may also comprise electrodes for acquiring signals of a non-physiological type and/or connections to sensors, transducers, and devices that are not integrated with the fabric.

### Brief Description of the Drawings

Further characteristics and advantages of the invention will become better apparent from the following detailed description, given by way of non-limiting example and illustrated in the accompanying drawings, wherein:
Figure 1 is a schematic view of a fabric that comprises conducting elements and non-conducting elements according to the present invention;
Figure 2 is a block diagram that illustrates schematically the components of a control device and of a data collection and post-processing system;
Figure 3 is a functional diagram of the overall circuit of an exemplifying embodiment of the control device according to the present invention as regards the collection of two biological signals, i.e., the electrocardiogram (ECG) and electrodermal activity (EDA);
Figure 4 is a flowchart illustrating the basic operation of the present invention;
Figure 5 is a flowchart that illustrates the steps for recognizing a valid contact between an electrode and the skin of the user according to a preferred embodiment;
Figure 6 is a flowchart that illustrates the steps for recognizing a valid signal according to a preferred embodiment.

### Ways of carrying_out the Invention

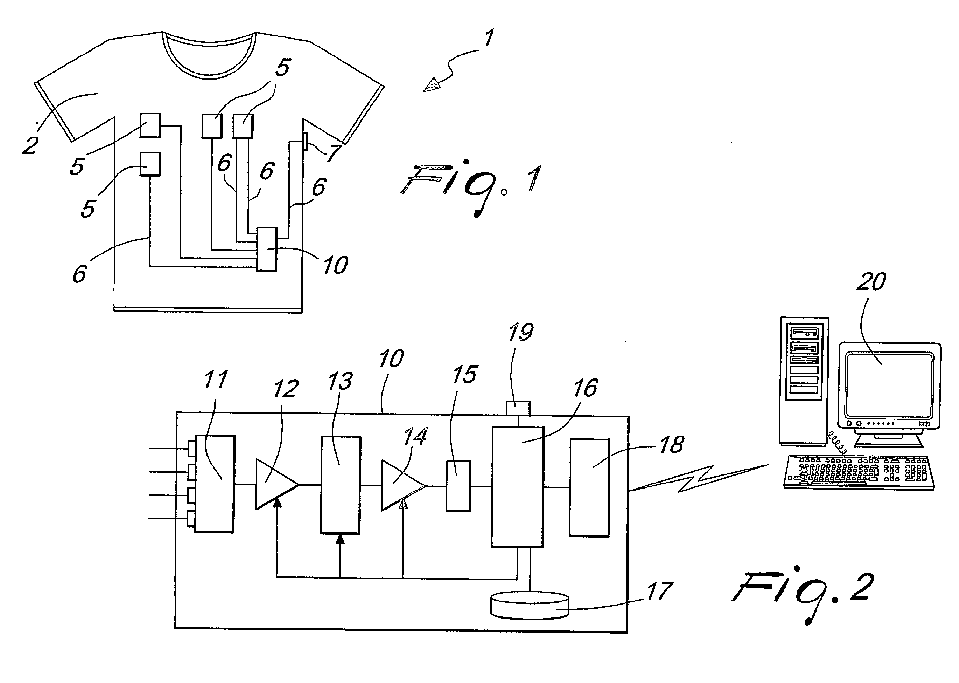

Figure 1 is a schematic view of the elements that compose the integrated structure 1 according to the present invention, and illustrates a fabric 2 that is shown in the form of an item of clothing, particularly in the form of an undershirt, onto which electrodes 5 are applied. Each electrode is connected at least to one first end of at least one conducting medium 6, the second end of which is in turn connected to a communications port 11, shown schematically in Figure 2, of a control device 10. The electrodes 5 are mainly sensors capable of acquiring a physiological signal that originates from the subject whose skin is in contact with them. However, they can also be actuation electrodes, for example capable of carrying current or microcurrent to the skin of the subject. In the present description, therefore, the term electrode is to be generically understood as an element capable of working in both directions, carrying signals from the patient to the control device 10 and from the control device to the patient. Depending on the circumstances, it is obvious for the person skilled in the art to understand whether the electrode 5 is acting as a sensor or as an actuator. The electrodes 5 may be of any kind, but are preferably obtained by weaving, spinning or sewing conducting fibers within the substrate of the fabric, so as to be monolithic with the fabric 2 in which they are inserted, or in any case are composed of conducting material, even of a different type. The electrodes may of course also be of a conventional type and may be applied to the item of clothing by affixing them, for example by means of adhesive or Velcro, so long as they are connected to the end of a conducting medium 6 for connection to the control device 10.

The electrodes 5 are arranged on the fabric 2 in predefined positions, depending on the final destination of the integrated structure 1, on the method of contact between the fabric and the skin of the user, and on the typical activity of said user.

Figure 1 further illustrates external sensors (and/or transducers) 7, which are connected in a fixed or detachable manner to one end of the conducting media 6. Such external elements are sensors and/or transducers of a generic type that are adapted to acquire signals of various kinds, such as temperature sensors, microphones and accelerometers. Instead of the external sensors 7, it is also possible to connect to the conducting media 6 more complex biomedical devices, which then send their information to the control device 10 and can be actuated and controlled by said device.

The conducting media 6 for connection between the electrodes 5 and the control device 10 are also preferably provided by spinning, sewing or weaving in the fabric 2 and are made of conducting material. The conducting media 6 are further preferably insulated one another and connect each electrode 5 to a different communications port 11 of the control device 10.

Moreover, the conducting elements are be insulated by means of locally removable material, for example insulating paint or enamel, so that by removing the insulating material of the conducting elements in specific regions it becomes possible, in these points, to acquire signals and provide electrical coupling with other conducting elements.

The control device 10, shown schematically in Figure 2, thus comprises a plurality of communications ports in the block 11, means 13 for preprocessing the signals received at the inputs of the communications ports 11, processing means 16 and a transceiver 18 for communication with external units 20. The device 10 may further comprise means 17 for storing data locally and interface means 19, for example in the form of buttons, for receiving commands from the user.

The control device 10 is preferably but not exclusively arranged on the fabric 2 by sewing or permanent or semi-permanent application so as to complete the integrated structure 1.

Moreover, the control device 10 can be fully or partially integrated with the fabric and rendered waterproof by way of conventional methods that are known in the prior art.

Operation of the device according to the invention is as follows. The user wears the integrated structure or, depending on the embodiment, otherwise places himself in contact with it so that at least some of the electrodes 5 arranged on the fabric 2 make contact with his skin in preset points. The electrodes 5 are redundant, and the signal that arrives from each electrode, in this case used as a sensor, can be weaker or stronger depending on the quality of the contact and on said position. Different signals, each originating from a different sensor, reach the communications ports 11 of the control device 10, which determines which of the received signals are to used in order to optimize the quality and reliability of the acquisition. For this purpose, the control device 10 initially utilizes the preprocessing means 13 and the configuration means 12, which comprise a plurality of channels for preconditioning the signal, which allow to filter the signals, accordingly allowing only part of the information to reach the processing means 16. Said preprocessing means, which initially act on the basis of default settings, modify their behavior under the guidance of the processing means 16, whose intelligence comprises an acquisition control system. The information required to drive and appropriately remap the signals in input to the communications ports 11 in order to achieve the intended goal are sent to the preprocessing means 13 and reconfiguration means 12. Redundancy of the input signals is achieved by a distributed arrangement of sensors and transducers 5 (also redundant) on the fabric 2 of the integrated structure 1.

The processing device 16 drives the reconfiguration of the preprocessing means 13 according to three different modes.

The first mode relates to the possibility to select, even autonomously on the basis of a prewired onboard intelligence, which signals to record or otherwise use among the signals that arrive from the electrodes 5, so as to allow selective monitoring only of the biological values of interest, for example cardiac signals.

The second mode relates to the characterization and selection of the most appropriate sensors for recording the selected signals that arrive from the electrodes 5. For example, assuming that three sensors have been arranged proximate to the heart of the user, it becomes possible to ignore the signals that are not qualitatively valid if one or more sensors 5 are not acquiring a signal of sufficient quality or intensity.

The third mode relates to the possibility to modify the signal acquisition settings dynamically during operation, both locally by the intervention of the user on the control device 10 and remotely with instructions that arrive from the external units 20.

Automatic reconfiguration by means of the electronic systems of the control device 10 of the inputs 11 allows to efficiently utilize the redundancy of the electrodes 5. The reconfiguration capability is therefore understood as a possibility to modify both the type of acquisition, for example from unipolar with neutral reference to differential, and the acquisition points, i.e., the sensors, for an identical acquisition mode, as well as the function of said electrode.

Therefore, as regards the reconfigurability of the system, the entire control device 10 according to the present invention operates according to two different modes, which however can be mutually integrated.

The signals that are most suitable for monitoring a given biological value, for example of the cardiac type, are selected by dynamically identifying the most appropriate sensors on the basis of the required monitoring mode and as a function of the condition of the subject. Simultaneously with this selection, the information that arrives from the electrodes that at the moment are not being used since they are not useful for acquisition are completely excluded automatically, i.e., are filtered upstream of the processing means, so as to prevent said sensors from acting as noise-gathering elements or as artifact generators.

In greater detail, the processing circuit 16 as shown in Figure 2 is internally provided with a microcontroller, DSP, FPGA and more generally programmable devices or a combination thereof, capable of modifying the internal connections between the communication ports 11 and the processing means located downstream by means of the selection blocks 12 and 14 on the basis of parameters obtained both from the physiological signals and from the signals that indicate the quality of the contact.

In particular, Figure 2 illustrates a block 12 that acts as a multiplexer and selects and carries the various signals received in input from the ports 11 to the already-mentioned preprocessing means 13, which eliminate the signals that are not specifically of interest, and to an additional block 14, which acts as a multiplexer and produces a data stream that is free of signals that are not of specific interest for the current application. The signal is then passed in input to the block 15, which substantially comprises an A/D converter for converting the signal from analog to digital. The signal is thus ready to be managed by the processing means 16, which further contain the intelligence for performing autonomous reconfiguration of the system. The signal is then transmitted by means of the transceiver 18 to external units, which can be electronic computers, plotters, printers and any other device for processing, storing or printing data. Alternatively or simultaneously, the signals can be stored in the memory means 17 and used, printed or transferred to external units at a later time.

The intelligence of the system can be programmed with conventional methods and techniques, with two different purposes: the first purpose is to reconfigure the acquisition system in order to obtain the maximum possible amount of information, i.e., receive as many signals as possible among those available in input to the communications ports 11; the second purpose is to reconfigure the acquisition system in order to achieve the longest possible optimum stability and quality of a single signal or in any case of a reduced set of signals.

Accordingly, the first criterion provides for selection among the various sensors in order to try to maintain the largest possible number of different signals in accordance with the constraints of quality and duration of the signal in a useful portion.

The second criterion can lead, for an equal number of sensors being used, to the provision of a single useful signal, or in any case of a reduced set of signals, in output from the acquisition and preprocessing chain. The remaining signals in input to the communications ports 11 in this case can be used to evaluate the stability and quality of the contact between the sensor that receives the useful signal or the sensors that receive the various useful signals, and the skin of the user. All the reconfigurability resources can therefore be dedicated to a single signal or to a reduced set of signals among the available signals, so as to optimize the use of the integrated structure according to the present invention even for the acquisition of specific and critical signals.

One reason why the control device controls a reconfiguration of the vector of the signals in input to the communications ports 11 is the possibility to utilize the multifunctionality of the sensors, which can be used both to acquire physiological signals of a different type and to acquire information related to the contact between the skin of the user and the regions of fabric, particularly of the sensors located in said regions, and finally for detecting noise and interference introduced by the current activity of the user.

Therefore, depending on the type of signal that one wishes to acquire, the control device 10, substantially by modifying the parameters of the filters, the preconditioning structure in the preprocessing means 13 and the processing of the block 16, is capable of modifying the operation of its processing network. The electrical signal that arrives from the sensors 5 can in fact be routed along different paths depending on the physiological information to be extracted. The methods for controlling and reconfiguring the signals in order to achieve these goals are autonomously managed by the control device 10, which is capable of modifying the flow and selection of the input and output signals both according to preset rules and according to rules that can be adapted to the type of intelligence being used, for example fuzzy logic or neural networks of a type that is known in the prior art. The term "intelligence" is therefore understood as a set of algorithms which, on the basis of thresholds, fuzzy sets or any other conventional interpretational model, even of the self-learning type, is capable of analyzing information and of deciding which actions to perform.

As regards the assessment of the quality of the contact between the sensor and the skin of the user and of the signal transmitted in input to the corresponding communications port 11, the control device substantially uses two kinds of criteria. A first criterion uses the multifunctionality of the sensors 5 and therefore utilizes the sensors for example to detect the electrode-skin coupling resistance by means of a processing that is different with respect to the processing for acquiring the physiological signal. The second criterion consists in analyzing in real time the received signal, comparing it with expected or known behaviors.

In the first case, the signal used to control the quality of the contact may also have its own information content, such as for example EDA (electrodermal activity). This measurement can be performed with polling operations, by modifying for short periods the configuration of the sensors, or can be superimposed on other signals.

The preprocessing system or directly the processing system 16 in this case provide band separation of the various signals depending on their content, so as to identify and correctly use the information mixed in the output signal.

When the control system verifies, by way of the intelligence that is present in the block 16, on the basis of preset rules or external commands that originate for example from external units 20, that there is a contact that is sufficient to acquire the intended signals, said processing means 16 drive the switching of the network, thus placing the operation of the system within the second type of cited method and therefore shifting to a real-time control of the acquired signal or signals.

The signal in input can be used in different manners, which are well-known in the prior art, to control the quality of the electrode/skin contact. In the preferred embodiment, the operation is performed by way of tests on the absolute threshold and on the derivative of the input signal. This method allows to establish the quality of the physical contact between the electrodes 5 and the skin of the user even by using a simple and unsophisticated microcontroller. In a different embodiment, the shape of the signal in input is instead evaluated by means of a DSP.

As previously mentioned, the choice of the parameters for monitoring can be also controlled remotely, by way of commands imparted from outside. In particular, remote reconfigurability can include modification by the physician, physiotherapist or the user himself of the physiological signals to be acquired and of the time intervals for their monitoring.

The person skilled in the art easily understands that an appropriately provided communications channel can be used for reconfiguration, and that it is instead possible to use non-dedicated channels by utilizing the methods known in the prior art for communication between systems.

The system can be reconfigured remotely, preferably by way of polling operations performed at regular intervals, toward one or more external units 20 provided with a suitable receiver, which recognizes the request and sends a new configuration, or by means of any other alternative form of communication suitable for the purpose, for example by means of transponders. The transceiver 18 may also prompt or request the sending of reconfiguration data by sending one or more characters or series of characters identified by the external unit 20 as a reconfiguration request.

The control device 10 according to the present invention ensures the electrical isolation of the non-useful fraction of the electrodes by way of conventional systems, such as analog multiplexers or buffers, and is therefore capable of placing said unused electrodes at a high impedance, also deactivating the corresponding electronic components, if necessary.

In view of the nature of the treated signals and of the users being monitored, the switching operations moreover do not require times that are particularly fast in electronic terms. The human system in fact has a very slow dynamic behavior from the standpoint of electronic circuits, on the order of tens or hundreds of milliseconds; therefore such dynamic behavior does not require particularly high-performance and particularly expensive electronic circuits, therefore allowing to obtain an economically advantageous product. Moreover, this inherent slowness of biological systems in general allows to dedicate more time to redundancy management and to control of the signal. The system, which has a conventional type of "intelligence" as defined above, is in fact capable of detecting whether the received signals are of a non-"physiological" nature, and therefore superfluous, and remedies them by differently reconfiguring the signal acquisition system.

The central core of reconfigurability is the possibility to acquire the contact and its quality in order to then control the analog multiplexers, as described above, or equivalent systems.

In a preferred embodiment, a number of biological values can be simultaneously measured by the same electrodes (for example, ECG and EDA). The information related to each value is then extracted by filtering the signal that arrives from the electrodes in the various bands of the signals, by way of example but not necessarily divided into the intervals 0-0.5 Hz for EDA and 0.5-125 Hz for ECG.

The EDA signal can also be used to verify the quality of the contact. In this case, in another preferred embodiment the EDA signal can also be measured by inverting for a few milliseconds the sensors used to acquire the ECG signal.

Operation of the control device 10 and of its components is now described in greater detail in an exemplifying embodiment, which provides the person skilled in the art with a clearer vision of the methods of embodiment of the invention. This embodiment is to be understood as being provided merely by way of illustration with no constraint in any way. It is in fact obvious for the person skilled in the art to understand that the components used can easily be replaced with other equivalents, all of which are within the scope of the inventive concept according to the invention.

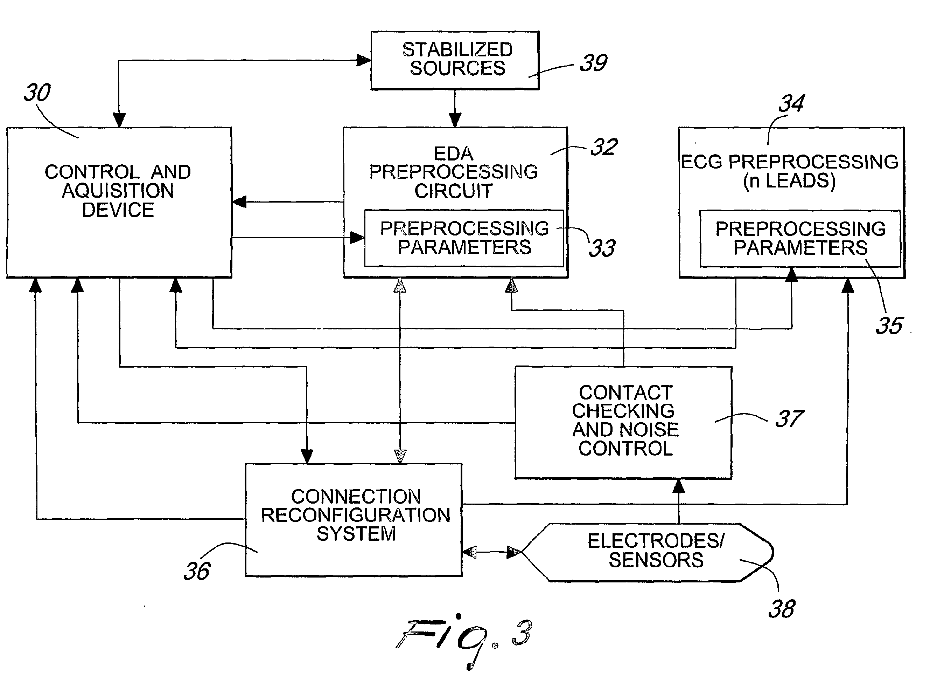

Figure 3 is therefore a view of an exemplifying embodiment of the entire system, in which the diagram of the transmission module 18 and of the memory block 19 has been omitted for the sake of clarity. In particular, the illustrated device 10 allows to measure ECG and EDA.

The control and acquisition unit is substantially constituted by a microcontroller 30, which deals with the management of the entire system, particularly with the selection of the various combinations of possible electrodes among the available ones. In view of the limited frequency band of the acquired signals, it is therefore possible to use relatively low sampling frequencies for each channel and therefore for example it is possible to use a serial ADC, optionally with pipelining, for signal acquisition.

The signals acquired by the ADCs provided internally or externally with respect to the microcontroller 30, preprocessed by means of the EDA preprocessing circuit 32 and of the ECG preprocessing circuit 34, are analyzed by said microcontroller, which by means of a control based on the derivative and amplitude of the signals, which have known behaviors in physiological signals of good quality, can easily identify critical situations.

Furthermore, the EDA signal itself provides a direct indication of the quality of the contact between the electrode and the skin.

It is possible to perform EDA measurement simultaneously with the ECG signal by using the same electrodes and configuring the preprocessing circuits 32 and 34 according to typical parameters by means of the appropriately provided structures of the blocks 33 and 35 provided in said preprocessing circuits described above.

There are some cases in which this operation cannot or must not be performed. In particular, indeed in the circumstance in which the signal of interest is deemed to be of good quality, the system may automatically disable this control.

Moreover, Figure 3 illustrates stabilized current sources 39 for measuring EDA. By increasing the injected current by a few µA, always within the safety limits for the user, it is thus possible to amplify the measured signal.

Thanks to the EDA acquisition circuit it is also possible to acquire the noise, in order to try to compensate for system drifts and system noise both at low frequency and at frequencies within the band of the ECG signal.

Other signals that arrive from sensors and transducers 7 of another type, for example accelerometers received in input from the system to the block 38, are routed to the control unit through an ADC or directly in digital form if the signal is already provided in this form, and can be activated or deactivated according to the selected configuration. In particular, the device has a reconfiguration block 36 and a noise and contact control block 37, whose operation, already described, is clarified even further in the description that follows.

The information that arrives from other sensors and transducers, which therefore do not belong to the set of electrodes that carry the signal used to evaluate EDA and ECG, may also be used to identify situations of potential instability. For example, the information that arrives from the accelerometers can provide indications regarding any physical activity performed by the user and therefore alert the system regarding a higher risk of instability in electrode-skin contact.

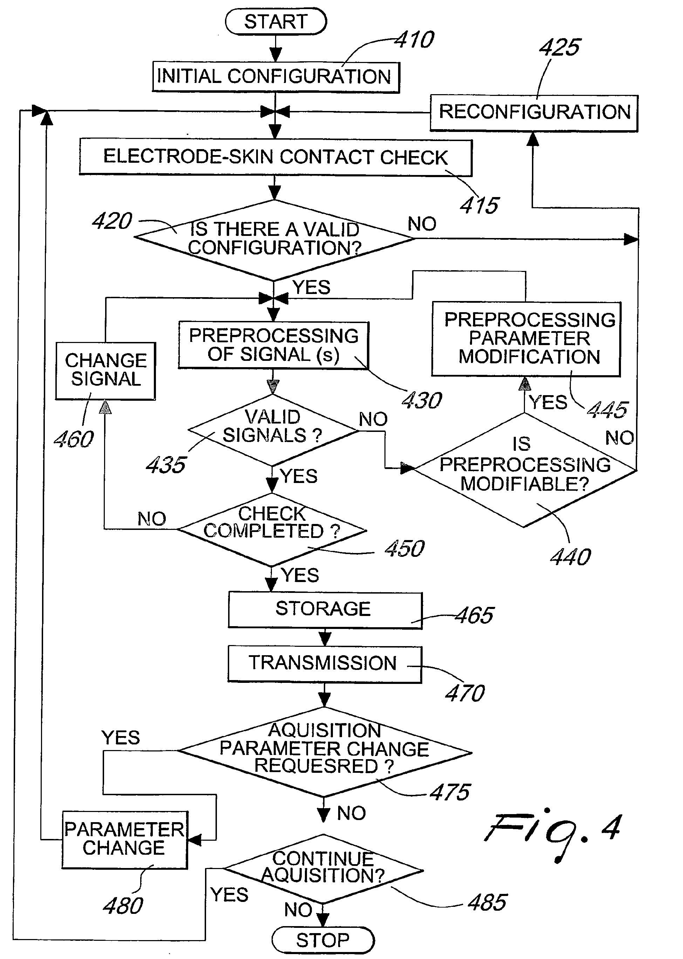

With reference to Figures 4, 5 and 6, the operation of the system is now described further with reference to a preferred and non-limiting exemplifying embodiment, which further clarifies the inventive concept on which the present invention is based.

In particular, with reference to Figure 4, in step 410 the device 10 is initialized to an initial configuration. Said initial configuration consists substantially of a selection performed by an operator, for example a physician or the user himself who is wearing the item of clothing. To continue with the example of the electrocardiogram, the physician can set a configuration that requires the system to automatically select five electrodes, for example three on the left and two on the right of the body of the patient.

In step 415, a sequence for controlling the electrode-skin contacts is preformed in order to identify a valid configuration, i.e., a configuration in which five electrodes selected within the set applied to the body of the patient are ensuring good contact with the skin of the patient.

The system then selects a first n-ple of electrodes and verifies the quality of the contacts of each electrode. If the outcome of this test, performed in step 420, is negative, i.e., not all the electrodes of the n-ple meet the requirements, reconfiguration operations are performed (step 425) and the cycle resumes with the analysis of the next n-ple.

When the device identifies a valid configuration, the procedure for preprocessing the signal or signals is activated in step 430, verifying the quality of the physiological values extracted from the involved electrodes (step 435). The signal acquired from each electrode, despite the good electrode-skin contact, may in fact be disturbed by the noise collected during its travel to the communications ports provided in the reconfiguration system 36 and by other physiological signals that are not of interest.

If the signals involved are not valid, the device checks in step 440 the possibility to further modify the type of signal preprocessing, for example by setting differently the parameters of certain filters. In this case, in step 445 the preprocessing parameters are modified and signal preprocessing is performed again (step 430).

If instead it is not possible to identify a further processing reconfiguration possibility, it becomes necessary to reconfigure the system again and therefore flow returns to the step 425.

When the outcome of the test in block 435 is positive and the signal or signals involved are valid, step 450 checks whether all the signals of interest have been checked. If the result is negative, the next signal or signals are selected (step 460) and the cycle resumes with the preprocessing process in step 430.

Once checking of all the involved signals has been completed, said signals are stored (step 465) and/or transmitted to external units (step 470).

The device then checks, in step 475, whether there is a pending request to modify the acquisition parameters that originates from outside or from the control system. If there is, the new parameters are modified in step 480. If there is not, in block 485 the system checks whether the acquisition process must be continued or suspended, for example due to a pending shutdown request originating from outside or under the control of a timer. Depending on the circumstances, therefore, activity resumes from step 415 or ends.

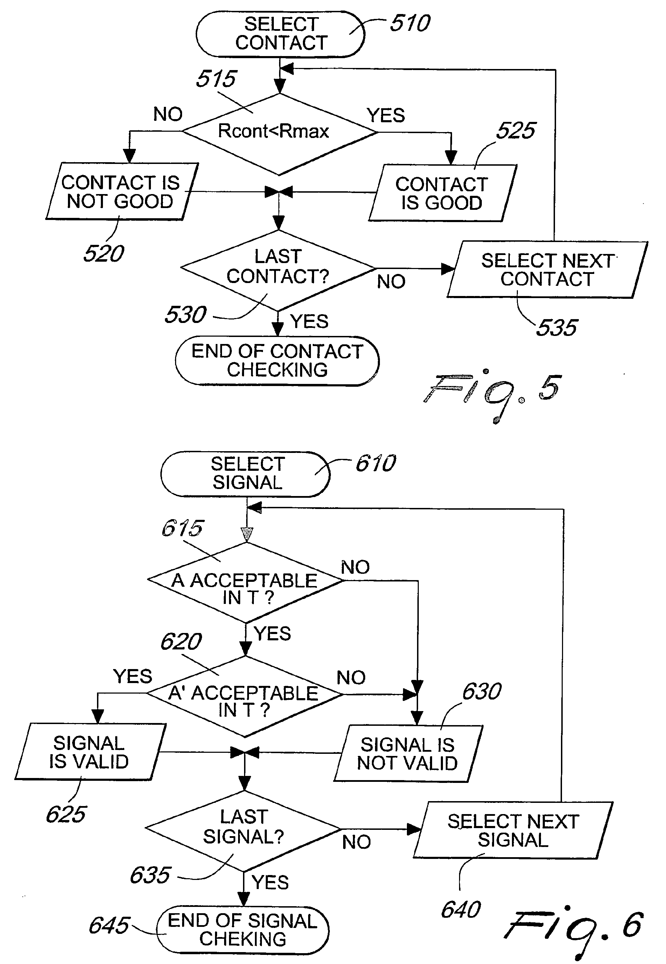

With reference to step 415 described above and to the block 37 of Figure 3, the steps for recognizing a valid contact between an electrode and the skin of the user according to a preferred embodiment are shown in greater detail in Figure 5.

In step 510, a first contact defined by an n-ple of electrodes is selected, and the corresponding electrode-skin contact resistance R_{cont} is acquired. If (step 515) in a time slot T this value exceeds a maximum limit Rₘₐₓ for a significant time, control moves to step 520, since the contact is not valid. Otherwise, flow proceeds to the block 525. Step 530 checks whether all the n-ples of electrodes have been checked.

If they have not, step 535 selects the next n-ple and the cycle resumes form step 515; otherwise, contact checking ends with a positive outcome.

With reference to the block 435 of Figure 4, a flowchart illustrating the steps for recognizing a valid signal according to a preferred embodiment is shown in Figure 6.

Step 610 selects a first signal, understood as an extracted biological value, acquires its amplitude A and extracts the derivative A' thereof in a time slot T that is appropriately sized according to the type of signal to be acquired. If said amplitude, checked in step 615, exceeds a maximum limit Aₘₐₓ for a significant time, the signal is rejected in step 630 because it is not valid. Step 635 checks whether all the available signals have already been checked. If they have not, step 640 selects the next signal and the cycle resumes from step 615.

If in step 615 the signal complies with the defined amplitude limits, flow continues in step 620, in which the derivative and its behavior are compared with the reference values and with the values acquired on the same signal in a convenient previous time interval. If the derivative is not acceptable, the signal is again marked as not valid in step 630. Otherwise, the signal is marked as valid in step 625. In both cases, flow advances to the test 635 and the cycle is repeated until all the signals have been checked. In output from the block 645, therefore, the device according to the invention knows the validity status of the current configuration.

Optionally, after the step 620 and if the expected type of signal has an expected form in the selected time interval, it is possible to provide another step that checks whether the shape of the acquired signal coincides with the expected shape. In this case also, if the test is negative, the signal is marked as not valid.

Once the electrodes that provide a valid electrode-skin contact have been selected and the set of signals of sufficient quality derived from said electrodes has been selected, it is thus possible to monitor the information acquired by the the activated sensors.

As already mentioned, the acquired information can be integrated with information that arrives from external sensors. The information that arrives from the external sensors/transducers can constitute independent information to be monitored and recorded. This information may also be used to process the signals that arrive from the integrated sensors. For example, the data that arrive from an accelerometer quantify the movement of the user. This information can be used for monitoring but it can also be used to alert the system to the risk of instability in skin-electrode contact.

The function of the various electrodes 5 and 7 may of course be any and may be used for the most disparate purposes wherever the acquisition of certain biological parameters of the user can provide information sufficient to determine correct monitoring. For example, it is possible to constantly monitor the heartbeat rate by arranging contact electrodes in the points usually used for this purpose, and to use physical principles that are known in the inventive context defined herein. An example is given by the operating principle of thermocouples. By applying two conducting elements 6 constituted by suitable different metals and insulated with the remainder of the path, each ending in a different input in the communications ports 11, it is possible to determine the potential difference between the two input points and, by way of known principles, determine the temperature of the skin in contact with the junction point of the two conducting elements. By using a battery of thermocouples thus provided and arranging the junctions in different points of the item of clothing, it is thus possible to define a thermal map of the user.

It has thus been shown that the present invention achieves the intended aim and objects. In particular, it has been shown that the method and system described allow to integrate physiological signal acquisition functionalities in garments, items of clothing or apparel that allow to perform monitoring even for long periods of time without introducing the traditional inconvenience factors in users to which the integrated structure according to the present invention is applied. It has also been shown that by the redundancy of the sensors, their multifunctionality and an adequate control and processing device it is also possible to adequately reconfigure the integrated structure so as to optimize signal acquisition and assess the quality of the contact between the electrode and the skin of the patient, activating in each instance the inputs of the signals acquired from different sensors arranged in the fabric of the integrated structure which ensure the best quality of the monitoring action.

The invention may also be conveniently an integral part of a more complex system that allows to control external elements for the administration or the activation of drugs to the patient in various forms, in accordance with the administration or activation techniques that are typical of a given drug. Moreover, it is evident that it can also be used to control systems that apply a physical value to the user, for example to control a defibrillator. The scope of protection is defined by the appended claims.

## Claims

1. An integrated structure (1) for acquiring physiological signals, comprising a fabric (2) composed of conducting fibers and non-conducting fibers,
multiple electrodes (5) provided by means of said conducting fibers;
multiple conducting elements (6), insulated one another, for connection between said electrodes (5) and inputs of a control device (10), said control device (10) comprising means for selective processing of electrical signals that originate from said electrodes (5), the conductive elements (6) are **characterized in that** insulated by virtue of locally removable material, so as to allow electrical coupling between two or more of said conducting elements (6) and between the conducting elements (6) and the skin of a user.

2. The integrated structure according to claim 1, **characterized in that** a plurality of said electrical signals is of a biological kind.

3. The integrated structure according to claim 1, **characterized in that** a plurality of said electrical signals is of a non-biological kind.

4. The integrated structure according to claim 1, **characterized in that** a plurality of said conducting elements (6) connects the control device to sensors, actuators or external devices.

5. The integrated structure according to claim 1, **characterized in that** said electrodes (5) can be applied equally to the skin of a user or to the conducting surface of conventional electrodes which are in turn applied to the skin of a user.

6. The integrated structure according to claim 1, **characterized in that** said control device (10) further comprises means for sending electrical signals selectively to said electrodes (5).

7. The integrated structure according to any one of the preceding claims, **characterized in that** said conducting elements are selected from the group that comprises:
- conducting fibers spun within said fabric;
- conducting fibers arranged on said fabric by affixing, preferably by gluing, embroidering, stitching;
-- conducting fibers inserted within sheaths or similar structures formed within the fabric or with the fabric.

8. The integrated structure according to any one of the preceding claims, **characterized in that** said conducting elements (6) are constituted by conducting materials that can also be mutually different.

9. The integrated structure according to any one of the preceding claims, **characterized in that** each electrode (5) is suitable to detect and send a plurality of signals of different origin.

10. The integrated structure according to the preceding claims, **characterized in that** each one of said electrodes (5)
can be selected, even dynamically, from a wider group of available electrodes;
can be configured, even dynamically, so as to be able to acquire and send signals of a different kind.

11. The integrated structure according to claim 10, **characterized in that** said control device (10) comprises means for performing operations for configuring and selecting said electrodes (5) autonomously according to preset programs.

12. The integrated structure according to claim 10, **characterized in that** said control device (10) comprises externally driven means for performing operations for configuring and selecting said electrodes (5).

13. The integrated structure according to any one of the preceding claims, **characterized in that** it is configured in the form of an item of clothing (2).

14. The integrated structure according to any one of the preceding claims, **characterized in that** it is configured in the form of a generic fabric that is arranged within the environment and with which a user can make contact.

15. The integrated structure according to claim 14, **characterized in that** said generic fabric is part of a sheet, a tablecloth, a sofa cover, a rest support, a steering wheel cover.

16. The integrated structure according to any one of the preceding claims, **characterized in that** said plurality of electrodes (5) comprises electrodes for acquiring non-biological signals.

## Patentansprüche

1. Integrierte Struktur (1) zum Erfassen physiologischer Signale, die ein Gewebe (2) aufweist, das aus leitenden Fasern und nichtleitenden Fasern besteht, mehrere Elektroden (5), die mittels der leitenden Fasern zur Verfügung gestellt sind; mehrere leitende Elemente (6), die voneinander isoliert sind, zur Verbindung zwischen den Elektroden (5) und Eingängen einer Steuervorrichtung (10), wobei die Steuervorrichtung (10) eine Einrichtung zum selektiven Verarbeiten elektrischer Signale aufweist, die von den Elektroden (5) entstehen, **dadurch gekennzeichnet, dass** die leitenden Elemente (6) mittels lokal entfernbaren Materials isoliert sind, um eine elektrische Kopplung zwischen zwei oder mehreren der leitenden Elemente (6) und zwischen den leitenden Elementen (6) und der Haut eines Anwenders zuzulassen.

2. Integrierte Struktur nach Anspruch 1, **dadurch gekennzeichnet, dass** eine Vielzahl der elektrischen Signale von einer biologischen Art ist.

3. Integrierte Struktur nach Anspruch 1, **dadurch gekennzeichnet, dass** eine Vielzahl der elektrischen Signale von einer nicht biologischen Art ist.

4. Integrierte Struktur nach Anspruch 1, **dadurch gekennzeichnet, dass** eine Vielzahl der leitenden Elemente (6) die Steuervorrichtung mit Sensoren, Stellgliedern oder externen Vorrichtungen verbindet.

5. Integrierte Struktur nach Anspruch 1, **dadurch gekennzeichnet, dass** die Elektroden (5) gleichermaßen an die Haut eines Anwenders oder an die leitende Oberfläche von herkömmlichen Elektroden, die wiederum an die Haut eines Anwenders angelegt sind, angelegt werden können.

6. Integrierte Struktur nach Anspruch 1, **dadurch gekennzeichnet, dass** die Steuervorrichtung (10) weiterhin eine Einrichtung zum selektiven Senden elektrischer Signale zu den Elektroden (5) aufweist.

7. Integrierte Struktur nach einem der vorangehenden Ansprüche, **dadurch gekennzeichnet, dass** die leitenden Elemente aus der Gruppe ausgewählt sind, die folgendes aufweist:
- leitende Fasern, die innerhalb des Gewebes eingesponnen sind;
- leitende Fasern, die an dem Gewebe durch Befestigen, vorzugsweise durch Kleben, Sticken, Nähen, angeordnet sind;
- leitende Fasern, die innerhalb von Hüllen oder ähnlichen Strukturen eingefügt sind, die innerhalb des Gewebes oder mit dem Gewebe ausgebildet sind.

8. Integrierte Struktur nach einem der vorangehenden Ansprüche, **dadurch gekennzeichnet, dass** die leitenden Elemente (6) durch leitende Materialien gebildet sind, die auch wechselseitig unterschiedlich sein können.

9. Integrierte Struktur nach einem der vorangehenden Ansprüche, **dadurch gekennzeichnet, dass** jede Elektrode (5) dazu geeignet ist, eine Vielzahl von Signalen eines unterschiedlichen Ursprungs zu detektieren und zu senden.

10. Integrierte Struktur nach den vorangehenden Ansprüchen, **dadurch gekennzeichnet, dass** jede der Elektroden (5)
aus einer breiteren Gruppe verfügbarer Elektroden, sogar dynamisch, ausgewählt werden kann;
sogar dynamisch konfiguriert werden kann, um Signale unterschiedlicher Art erfassen und senden zu können.

11. Integrierte Struktur nach Anspruch 10, **dadurch gekennzeichnet, dass** die Steuervorrichtung (10) eine Einrichtung zum Durchführen von Operationen zum Konfigurieren und Auswählen der Elektroden (5) autonom gemäß voreingestellter Programme aufweist.

12. Integrierte Struktur nach Anspruch 10, **dadurch gekennzeichnet, dass** die Steuervorrichtung (10) eine extern angetriebene Einrichtung zum Durchführen von Operationen zum Konfigurieren und Auswählen der Elektroden (5) aufweist.

13. Integrierte Struktur nach einem der vorangehenden Ansprüche, **dadurch gekennzeichnet, dass** sie in der Form eines Elements einer Kleidung (2) konfiguriert ist.

14. Integrierte Struktur nach einem der vorangehenden Ansprüche, **dadurch gekennzeichnet, dass** sie in der Form eines allgemeinen Gewebes konfiguriert ist, das innerhalb der Umgebung angeordnet ist und mit welchem ein Anwender einen Kontakt herstellen kann.

15. Integrierte Struktur nach Anspruch 14, **dadurch gekennzeichnet, dass** das allgemeine Gewebe ein Teil eines Lakens bzw. Betttuchs, eines Tischtuchs, einer Sofaabdeckung, einer Ruhestütze, einer Lenkradabdeckung ist.

16. Integrierte Struktur nach einem der vorangehenden Ansprüche, **dadurch gekennzeichnet, dass** die Vielzahl von Elektroden (5) Elektroden zum Erfassen nicht biologischer Signale aufweist.

## Revendications

1. Structure intégrée (1) pour faire l'acquisition de signaux physiologiques, comprenant un tissu (2) composé de fibres conductrices et de fibres non conductrices, des électrodes multiples (5) créées au moyen desdites fibres conductrices ; des éléments conducteurs multiples (6), isolés les uns des autres, pour être connectés entre lesdites électrodes (5) et des entrées d'un dispositif de commande (10), ledit dispositif de commande (10) comprenant des moyens pour traiter sélectivement des signaux électriques provenant desdites électrodes (5), **caractérisée en ce que** les éléments conducteurs (6) sont isolés par un matériau amovible localement, de façon à permettre un couplage électrique entre deux ou plusieurs desdits éléments conducteurs (6) et entre les éléments conducteurs (6) et la peau d'un utilisateur.

2. Structure intégrée selon la revendication 1, **caractérisée en ce qu'**une pluralité desdits signaux électriques est de type biologique.

3. Structure intégrée selon la revendication 1, **caractérisée en ce qu'**une pluralité desdits signaux électriques est de type non biologique.

4. Structure intégrée selon la revendication 1, **caractérisée en ce qu'**une pluralité desdits éléments conducteurs (6) connectent le dispositif de commande à des capteurs, actionneurs ou dispositifs externes.

5. Structure intégrée selon la revendication 1, **caractérisée en ce que** lesdites électrodes (5) peuvent être appliquées de façon égale à la peau d'un utilisateur ou à la surface conductrice d'électrodes conventionnelles qui sont elles-mêmes appliquées à la peau d'un utilisateur.

6. Structure intégrée selon la revendication 1, **caractérisée en ce** ledit dispositif de commande (10) comprend en outre des moyens pour envoyer sélectivement des signaux électriques auxdites électrodes (5).

7. Structure intégrée selon l'une quelconque des revendications précédentes, **caractérisée en ce que** lesdits éléments conducteurs sont sélectionnées à partir d'un groupe qui comprend :
- des fibres conductrices liées dans ledit tissu ;
- des fibres conductrices disposées sur ledit tissu par fixation, de façon préférentielle par collage, broderie, piquage ;
- des fibres conductrices insérées dans des gaines ou des structures similaires formées dans le tissu ou avec le tissu.

8. Structure intégrée selon l'une quelconque des revendications précédentes, **caractérisée en ce que** lesdits éléments conducteurs (6) sont constitués de matériaux conducteurs qui peuvent être différents les uns des autres.

9. Structure intégrée selon l'une quelconque des revendications précédentes, **caractérisée en ce que** chaque électrode (5) convient pour détecter et envoyer une pluralité de signaux d'origines différentes.

10. Structure intégrée selon l'une quelconque des revendications précédentes, **caractérisée en ce que** chacune desdites électrodes (5) peut être choisie, même de façon dynamique, à partir d'un plus grand groupe d'électrodes disponibles ; peut être configurée, même de façon dynamique, de façon à ce qu'elle soit capable de faire l'acquisition et d'envoyer des signaux d'une sorte différente.

11. Structure intégrée selon la revendication 10, **caractérisée en ce que** ledit dispositif de commande (10) comprend des moyens pour effectuer des opérations de configuration et de sélection desdites électrodes (5) de façon autonome selon des programmes prédéfinis.

12. Structure intégrée selon la revendication 10, **caractérisée en ce que** ledit dispositif de commande (10) comprend des moyens pilotés de façon externe pour effectuer des opérations de configuration et de sélection desdites électrodes (5).

13. Structure intégrée selon l'une quelconque des revendications précédentes, **caractérisée en ce qu'**elle est configurée dans la forme d'un article vestimentaire (2).

14. Structure intégrée selon l'une quelconque des revendications précédentes, **caractérisée en ce qu'**elle est configurée dans la forme d'un tissu générique qui est disposé dans l'environnement et avec lequel un utilisateur peut entrer en contact.

15. Structure intégrée selon la revendication 14, **caractérisée en ce que** ledit tissu générique est une partie d'une feuille, d'une nappe, d'une housse de fauteuil, d'un support de repos, d'une couverture de volant de direction.

16. Structure intégrée selon l'une quelconque des revendications précédentes, **caractérisée en ce que** ladite pluralité d'électrodes (5) comprend des électrodes pour faire l'acquisition de signaux non-biologiques.
